(19) Europäisches Patentamt
European Patent Office
Office européen des brevets

(11) Numéro de publication: 0 238 408 B1

# (12) FASCICULE DE BREVET EUROPEEN

(45) Date de publication du fascicule du brevet: **06.12.89**

(51) Int. Cl.⁴: **C07C 9/04,** C07C 1/04, B01J 27/047

(21) Numéro de dépôt: **87400578.8**

(22) Date de dépôt: **16.03.87**

(54) **Procédé de production du méthane à l'aide d'un catalyseur thiorésistant et catalyseur pour la mise en oeuvre de ce procédé.**

(30) Priorité: **17.03.86 FR 8603761**

(43) Date de publication de la demande: **23.09.87 Bulletin 87/39**

(45) Mention de la délivrance du brevet: **06.12.89 Bulletin 89/49**

(84) Etats contractants désignés: **AT BE CH DE ES GB GR IT LI LU NL SE**

(56) Documents cités:
**US-A- 2 192 125**
**US-A- 3 429 679**

(73) Titulaire: **GAZ DE FRANCE, 23, rue Philibert Delorme, F-75017 Paris(FR)**

(72) Inventeur: **d'Emmerez de Charmoy, Roger Dennisson, 7 Impasse Saint-Maur, F-94700 Maisons-Alfort(FR)**
Inventeur: **Mari, Daniel, 47 rue de Sèvres, F-92100 Boulogne(FR)**
Inventeur: **Dalmon, Jean Alain, 5 Avenue de Général de Gaulle, F-69300 Caluire(FR)**
Inventeur: **Turlier, Pierre, 29 A, rue de Montriblond, F-69009 Lyon(FR)**
Inventeur: **Martin, Guy Antonin, 10 rue César Paulet, F-69660 Colonges(FR)**

(74) Mandataire: **Durand, Yves Armand Louis et al, Cabinet Z. Weinstein 20, Avenue de Friedland, F-75008 Paris(FR)**

EP 0 238 408 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention a essentiellement pour objet un procédé de production du méthane ou d'un mélange de gaz comprenant du méthane à l'aide d'un catalyseur résistant au soufre ou thiorésistant.

Elle vise également le catalyseur pour la mise en oeuvre de ce procédé.

La synthèse du méthane ou méthanisation constitue une étape déterminante dans le processus de production de gaz naturel de substitution (G.N.S) faisant appel à l'oxyvapogazéification du charbon.

Dans les procédés classiques de méthanisation, la réaction mise en jeu peut être représentée par l'équation :

$$CO + 3H_2 \longrightarrow CH_4 + H_2O \qquad (I)$$

$$\Delta H = -206,3 \text{ kJ/mole}$$

D'un point de vue industriel, pour réaliser cette synthèse, on utilise des catalyseurs, notamment à base de nickel.

Cependant, une telle synthèse industrielle souffre de nombreux inconvénients.

C'est ainsi qu'il est nécessaire d'adapter le rapport $H_2/CO$ du gaz à méthaniser à la valeur 3 requise par la stoechiométrie de la réaction précitée.

Par ailleurs, il est nécessaire d'éliminer les impuretés du gaz jusqu'à des niveaux résiduels très faibles d'environ moins de 0,1 ppm pour le soufre par exemple, ce qui nécessite des systèmes d'épuration très performants, et donc très coûteux.

Enfin, il est nécessaire, en raison de la très forte exothermicité de la réaction, de prévoir des dispositifs d'évacuation de la chaleur très performants et également coûteux.

En conséquence, la mise en oeuvre des procédés classiques de méthanisation dans une chaîne de traitement du gaz impose une séquence complexe qui affecte à la fois le coût des investissements nécessaires à la réalisation d'une installation et le rendement de transformation.

C'est pourquoi depuis quelques années on a cherché à développer de nouveaux catalyseurs résistants aux composés soufrés, qui permettraient une réduction des coûts d'investissement et de production du gaz final.

En effet, le mécanisme réactionnel observé, lorsqu'on utilise des catalyseurs thiorésistants est très légèrement différent de celui représenté par l'équation I.

Dans ce cas en effet, la méthanisation est dite directe, et la réaction chimique correspondante peut être représentée par l'équation :

$$2CO + 2H_2 \longrightarrow CH_4 + CO_2 \qquad (II)$$

$$\Delta H = -123,7 \text{ kJ/mole.}$$

La mise au point de catalyseurs thiorésistants est par conséquent tout à fait avantageuse puisqu'elle permet de s'affranchir des systèmes d'épuration complexes utilisés dans le cas des procédés classiques, et le mélange de gaz de départ présentant une composition proche de celle des gaz issus des générateurs de gaz de synthèse, on peut opérer sur un gaz brut non traité, après élimination des particules solides, et sans désulfuration préalable.

On évite ainsi une étape supplémentaire d'adaptation du rapport $H_2/CO$ évoqué pour les procédés classiques, car la plupart des procédés de gazéification du charbon conduit, en effet, à un gaz dont le rapport molaire $H_2/CO$ est voisin de 1.

En conséquence, l'utilisation des catalyseurs thiorésistants permet une simplification de la chaîne de traitement du gaz et offre une plus grande souplesse d'exploitation, en comparaison des procédés classiques de méthanisation. De plus, en raison de spécifications moins sévères pour la réaction, on obtient un gain sur le rendement de transformation qui peut atteindre jusqu'à 6%.

On a déjà proposé dans le document US-P 3 429 679 l'emploi de sulfure de tungstène comme catalyseur de méthanisation, mais ce catalyseur n'est pas utilisé avec un mélange de départ dans lequel le rapport molaire $H_2/CO$ est sensiblement égal à 1.

Par ailleurs, on a proposé dans le document US-P 2 192 125 de préparer du sulfure de tungstène par décomposition à la chaleur d'un composé répondant à la formule $(NH_4)_2WS_4$. Mais les conditions de préparation et notamment de température ne permettent pas de réaliser un catalyseur apte à la production de méthane avec une sélectivité élevée en méthane produit.

Les catalyseurs susceptibles d'activer la réaction de synthèse (II) précitée sont généralement des sulfures, notamment de molybdène, et généralement supportés.

C'est ainsi que l'on connait, notamment par le document US-P 4 260 553 des catalyseurs à base de molybdène, et plus généralement de vanadium ou tungstène, et des supports très divers tels que notamment oxyde d'aluminium, de silicium, de bore, de cérium, de titane, de zirconium, dont la concentration peut varier entre 0 et 1.

Cependant de tels catalyseurs présentent des performances limitées, en particulier sur le plan de leur sélectivité en faveur de la production de méthane.

Aussi, la présente invention a pour but de remédier à ces inconvénients en proposant un procédé de production du méthane à l'aide d'un catalyseur thiorésistant susceptible de donner d'excellents résultats, en particulier sur le plan de la sélectivité en méthane produit.

A cet effet, l'invention a pour objet un procédé de production de méthane ou d'un mélange de gaz comprenant du méthane à partir d'un mélange de départ qui comprend notamment du monoxyde de carbone, de l'hydrogène et des composés soufrés et que l'on met en contact avec un catalyseur thiorésistant à base de sulfure de tungstène, caractérisé en ce que ledit catalyseur présentant une surface spécifique comprise entre 20 et 100 $m^2/g$ est mis en contact avec ledit mélange de départ dans lequel le rapport molaire hydrogène/monoxyde de carbone

est sensiblement égal à 1, avec une vitesse spatiale (v.v.h) comprise entre 1000 et 10 000 $h^{-1}$, à une pression comprise entre 1 et 50 bars et de préférence entre 25 et 35 bars, et à une temperature comprise entre 250°C et 650°C.

La présente invention est donc basée sur la découverte selon laquelle du sulfure de tungstène non supporté, d'une surface spécifique de 20 à 100 $m^2/g$ et utilisé dans un mélange de départ contenant notamment $H_2$ et CO suivant un rapport molaire égal à 1, apportait une amélioration sensible des performances des catalyseurs de l'art antérieur.

En particulier, le mélange de gaz de départ peut contenir des composés soufrés en teneurs pouvant aller jusqu'à 5% en poids de soufre ce qui permet de supprimer une étape préalable de désulfuration, et d'augmenter ainsi le rendement de transformation.

Suivant une autre caractéristique du procédé conforme à l'invention, dans le cas de réacteurs à lits fixes, la réaction est effectuée à une vitesse spatiale (v.v.h) comprise entre 1000 et 10 000 $h^{-1}$, et de préférence voisine de 6000 $h^{-1}$.

Suivant un mode de réalisation préféré de l'invention, le catalyseur à base de sulfure de tungstène est utilisé sous une forme pure en lit fluidisé, ce qui permet d'assurer une température uniforme dans le réacteur. En fait, les points chauds sont évités car le lit fluidisé permet une évacuation aisée des calories dégagées par la réaction, par les parois, dans les réacteurs de petite taille et au moyen de tubes de transfert immergés dans le réacteur dans les unités industrielles.

L'invention vise également un catalyseur pour la mise en œuvre du procédé répondant à l'une ou l'autre des caractéristiques indiquées ci-dessus.

L'invention sera mieux comprise, et d'autres caractéristiques et avantages de celle-ci apparaîtront plus clairement à la lecture de la description explicative qui va suivre, faite en référence aux exemples non limitatifs qui seront donnés ci-après, et illustrés par la figure unique annexée qui est une courbe représentant l'activité comparée en fonction du temps de divers catalyseurs selon l'art antérieur et selon l'invention.

Le catalyseur thiorésistant, conforme à la présente invention est réalisé à base de sulfure de tungstène ($WS_2$). Ce catalyseur est obtenu par exemple par chauffage à 300-600°C de tétrathiotungstate d'ammonium de formule $(NH_4)_2WS_4$, sous un courant d'hydrogène contenant du sulfure d'hydrogène. Dans un exemple, le composé précité a été chauffé progressivement avec une vitesse de 4°C/mn sous un courant d'hydrogène contenant 15% en volume d'$HS_2$, jusqu'à une température d'environ 500°C, puis maintenu 4 heures à cette température. Le sulfure de tungstène ainsi obtenu a été refroidi durant 1 heure environ, sous la même atmosphère, puis a été transféré dans un flacon étanche sous argon.

On a répété ce mode opératoire à des températures de sulfuration de 400°C et 600°C, et les sulfures ainsi obtenus présentaient une surface BET de 55$m^2/g$ et 35 $m^2/g$, respectivement.

Ensuite, on a procédé à un test catalytique pour comparer les performances d'un catalyseur à base de sulfure de tungstène, conforme à la présente invention, avec celle d'un catalyseur répondant à la formule $MoS_2$, sur un support d'oxyde de cérium, analogue à celui décrit dans le brevet américain No. 4 260 553.

Ce test catalytique, qui avait pour but de comparer les performances de ces catalyseurs en terme d'activité et de sélectivité dans la production du méthane a consisté à mettre en contact chacun des catalyseurs avec un mélange de réactifs comprenant du monoxyde de carbone, de l'hydrogène et du sulfure d'hydrogène sous une pression de 30 bars avec une vitesse spatiale (v.v.h) d'environ 6000 $h^{-1}$, à une température d'environ 405°C.

On a analysé les gaz de sortie par chromatographie en phase gazeuse, ce qui a permis de déterminer en particulier, pour les différents catalyseurs précités l'activité, exprimée en nombre de moles de monoxyde de carbone converties en méthane par unité de temps et par gramme de catalyseur, ainsi que la sélectivité en méthane définie par le rapport du nombre de moles de méthane formées au nombre total de moles d'hydrocarbure et de dioxyde de carbone formées. Cette sélectivité sera référencée ci-après $S_1$.

On a également indiqué la sélectivité en éthane définie comme le rapport du nombre de moles d'éthane formées à la somme des nombre de moles de méthane et d'éthane formées. Cette sélectivité sera référencée ci-après $S_2$.

Les produits obtenus sont essentiellement constitués de méthane ($CH_4$), d'éthane ($C_2H_6$),de propane ($C_3H_8$), de dioxyde de carbone ($CO_2$) de monoxyde de carbone (CO) et d'hydrogène non transformé, ainsi que d'eau et d'hydrocarbures supérieurs sous forme de traces.

Les réactions mises en jeu sont les suivantes :

$$2\,CO + 2\,H_2 \longrightarrow CO_2 + CH_4$$

$$CO + 3\,H_2 \longrightarrow CH_4 + H_2O$$

$$CO + H_2O \longrightarrow CO_2 + H_2$$

$$2\,CO + 5\,H_2 \longrightarrow C_2H_6 + 2H_2O$$

$$4\,CO + 3\,H_2 \longrightarrow C_2H_6 + 2\,CO_2$$

$$2n\,CO + \frac{m}{2}\,H_2 \longrightarrow CnHm + n\,CO_2$$

$$n\,CO + (\frac{m}{2}+n)\,H_2 \longrightarrow CnHm + n\,H_2O$$

Enfin, on a indiqué une troisième sélectivité $S_3$ définie par le rapport du nombre de moles de monoxyde de carbone converties en méthane au nombre total de moles de monoxyde de carbone converties. Il s'agit en fait d'un "rendement en méthane".

Le tableau 1 ci-après et la figure 1 illustrent les résultats obtenus sur les catalyseurs décrits précédemment. La figure 1 illustre en particulier l'évolution de l'activité et de la stabilité des trois catalyseurs étudiés, sur une période de temps correspondant à 20 à 60 heures de fonctionnement ou de travail du catalyseur. Sur cette courbe, on a représenté en ordonnées l'activité du catalyseur, exprimée en nombre de moles de monoxyde de carbone transformées en méthane par seconde et par gramme de catalyseur.

Le tableau 1 illustre plus particulièrement la sélectivité comparée à 350°C et 400°C, et en présence de 100 ppm de sulfure d'hydrogène, en méthane, du sulfure de tungstène obtenu par sulfuration à 600°C et du catalyseur de l'art antérieur $MoS_2/CeO_2$.

Tableau 1

| Catalyseurs | Sélectivités ($H_2S$ = 100 ppm) | | | | | |
|---|---|---|---|---|---|---|
| | 350°C | | | 400°C | | |
| | $S_1$ | $S_2$ | $S_3$ | $S_1$ | $S_2$ | $S_3$ |
| $MoS_2/CeO_2$ Art antérieur | 0,27 | 0,28 | 0,24 | 0,34 | 0,14 | 0,31 |
| $WS_2$ (600) La présente invention | 0,46 | 0,11 | 0,43 | 0,45 | 0,08 | 0,43 |

Le tableau 2 illustre plus particulièrement, à différentes températures et en présence de 0,5% volumique de sulfure d'hydrogène la sélectivité comparée en méthane du sulfure de tungstène obtenu par sulfuration à 400°C et du catalyseur de l'art antérieur.

Tableau 2

| Catalyseurs | Sélectivités ($H_2S$ = 0,5%) | | | | | | | | |
|---|---|---|---|---|---|---|---|---|---|
| | 350°C | | | 400°C | | | 450°C | | |
| | $S_1$ | $S_2$ | S3 | S1 | S2 | S3 | S1 | S2 | S3 |
| $MoS_2/CeO_2$ Art Antérieur | 0,29 | 0,27 | 0,24 | 0,43 | 0,10 | 0,40 | 0,45 | 0,06 | 0,44 |
| $WS_2$ (400°C) | 0,36 | 0,18 | 0,32 | 0,46 | 0,08 | 0,44 | 0,48 | 0,04 | 0,47 |

Les résultats donnés dans les tableaux 1 et 2, et représentés sur la figure 1 mettent clairement en évidence la supériorité du catalyseur à base de sulfure de tungstène conforme à l'invention, par rapport aux catalyseurs de l'art antérieur pour ce qui est de la sélectivité en méthane et de l'activité par unité de poids tandis qu'il n'y a pas de variation sensible de la stabilité de ces deux types de catalyseurs. Les stabilités, exprimées en pertes relatives d'activité de ces catalyseurs, sur la période de temps expérimentée restent en effet comparables (de l'ordre de 25 à 35 % pour la période de temps de 20 à 60 heures environ).

Les tableaux 1 et 2 montrent clairement que la sélectivité en méthane mesurée pour des catalyseurs conformes à l'invention est non seulement très supérieure à celle d'un catalyseur de l'art antérieur, mais très proche de la sélectivité théorique maximum qui est de 0,5.

Des expériences effectuées à pression atmosphérique, ou avec des teneurs en $H_2S$ plus élevées confirment ces observations.

On a également réalisé un certain nombre d'expériences donnant des résultats comparables avec des catalyseurs à base de sulfure de tungstène présentant une surface spécifique variant entre 20 et 100 $m^2/g$.

On notera que la surface BET après test catalytique est très voisine des valeurs initiales pour les deux sulfures de tungstène obtenus par sulfuration à 400°C et 600°C.

Il est également important de noter que les catalyseurs conformes à l'invention sont en outre très supérieurs aux catalyseurs de l'art antérieur en ce qui concerne la sélectivité $S_3$ qui représente comme on l'a vu un rendement en méthane.

Comme on l'a également vu, la réaction de méthanisation étant fortement exothermique, le catalyseur est employé de préférence en lit fluidisé.

On a donc réalisé suivant l'invention un procédé de méthanisation qui permet de récupérer des quantités importantes de méthane en utilisant un catalyseur présentant une activité et une stabilité comparables aux catalyseurs connus de l'art antérieur.

## Revendications

1. Procédé de production de méthane ou d'un mélange de gaz comprenant du méthane à partir d'un mélange de départ qui comprend notamment du monoxyde de carbone, de l'hydrogène et des composés soufrés et que l'on met en contact avec un catalyseur thiorésistant à base de sulfure de tungstène, caractérisé en ce que ledit catalyseur présentant une surface spécifique comprise entre 20 et 100 $m^2/g$ est mis en contact avec ledit mélange de départ dans lequel le rapport molaire hydrogène/monoxyde de carbone est sensiblement égal à 1, avec une vitesse spatiale (v.v.h) comprise entre 1000 et 10 000 $h^{-1}$, à une pression comprise entre 1 et 50 bars et à une température comprise entre 250 et 650°C.

2. Procédé selon la revendication 1, caractérisé en ce que la réaction est effectuée à une vitesse spatiale (v.v.h) sensiblement égale à 6000 $h^{-1}$.

3. Procédé selon la revendication 1 ou 2, caractérisé en ce que le catalyseur précité est utilisé sous une forme pure en lit fluidisé.

4. Catalyseur thiorésistant à base de sulfure de tungstène pour la mise en œuvre du procédé selon l'une des revendications 1 à 3 et du type obtenu par chauffage d'un composé répondant à la formule $(NH_4)_2WS_4$, caractérisé en ce que que ledit chauffage est effectué à une température comprise entre 300 et 600°C sous un courant d'hydrogène contenant du sulfure d'hydrogène.

## Patentansprüche

1. Verfahren zur Erzeugung von Methan oder eines Methan aufweisenden Gasgemisches ausgehend von einem Ausgangsgemisch, das insbesondere Kohlenmonoxyd, Wasserstoff und schwefelhaltige Verbindungen umfaßt und welches man mit einem wolframsulfidbasischen schwefelbeständigen Katalysator in Berührung bringt, dadurch gekennzeichnet, daß der besagte, eine zwischen 20 und 100 $m^2/g$ liegende spezifische Fläche aufweisende Katalysator mit dem besagten Ausgangsgemisch in Berührung gebracht wird, bei welchem das Wasserstoff/Kohlenmonoxyd Molverhältnis im wesentlichen gleich 1 ist, mit einer zwischen 1000 und 10 000 $h^{-1}$ liegenden Raumgeschwindigkeit (v.v.h.) bei einem zwischen 1 und 50 Bar liegenden Druck und bei einer zwischen 250 und 650°C liegenden Temperatur.

2. Verfahren nach Anspruch 1, dadurch gekennzeichnet, daß die Reaktion bei einer Raumgeschwindigkeit (v.v.h.) im wesentlichen von 6000 $h^{-1}$ durchgeführt wird.

3. Verfahren nach Anspruch 1 oder 2, dadurch gekennzeichnet, daß der vorgenannte Katalysator in reiner Form in einem Fließbett verwendet wird.

4. Wolframsulfidbasischer schwefelbeständiger Katalysator zur Durchführung des Verfahrens nach einem der Ansprüche 1 bis 3 und derjenigen Gattung in welcher er durch Erwärmung einer der Formel $(NH_4)_2$ $WS_4$, entsprechenden Verbindung erhalten wird, dadurch gekennzeichnet, daß die besagte Erwärmung bei einer zwischen 300 und 600°C liegenden Temperatur in einem wasserstoffsulfidhaltigen Wasserstoffstrom durchgeführt wird.

## Claims

1. Process for the production of methane or of a mixture of gases comprising methane from a starting mixture which comprises in particular carbon monoxide, hydrogen and sulphured compounds and which is contacted with a tungsten sulfide-based fire resistant catalyst, characterized in that the said catalyst having a specific surface area comprised between 20 and 100 $m^2/g$ is put into contact with the said starting mixture in which the hydrogen/carbon monoxide molar ratio is substantially equal to 1, with a spatial speed (v.v.h.) comprised between 1000 and 10 000 $h^{-1}$, at a pressure comprised between 1 and 50 bars and at a temperature comprised between 250 and 650°C.

2. Process according to claim 1, characterized in that the reaction is effected at a spatial speed (v.v.h.) substantially equal to 6000 $h^{-1}$.

3. Process according to claim 1 or 2, characterized in that the aforesaid catalyst is used in a pure form in a fluidized bed.

4. Tungsten sulfide-based fire resistant catalyst for carrying out the process according to one of the claims 1 to 3 and of the kind obtained by heating a compound corresponding to the formula $(NH_4)_2WS_4$, characterized in that the said heating is effected at a temperature comprised between 300 and 600°C in a flow of hydrogen containing hydrogen sulfide.

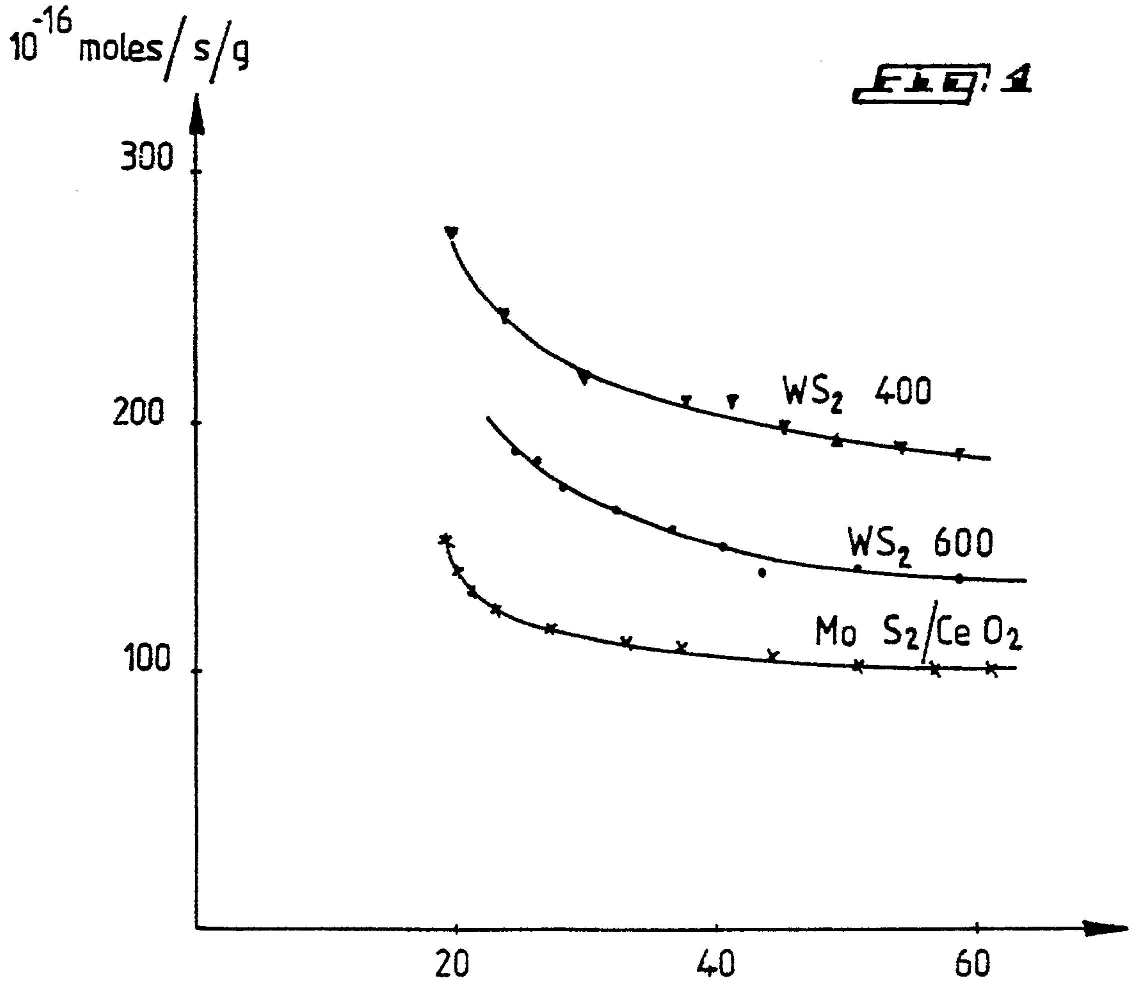


Fig. 1